# EUROPEAN PATENT APPLICATION

(11) **EP 0 865 764 A2**
(43) Date of publication of application: **23.09.1998**
(21) Application number: 98104744.2
(22) Date of filing: 17.03.1998
(51) Int. Cl.: A61B 5/20

(54) **Urinometer having a backflow-proof valve**

(30) Priority: 17.03.1997 IT MI970608
(71) Applicant: MEDINET S.p.A., I-20154 Milan (IT); Kendall Proclinics, S.L., 08160 Montmelo (Barcelona) (ES)
(72) Inventor: Alfonso Olivero, Juan Maria, 08008 Barcelona (ES); Apolet, Josef Berek, 20146 Milan (IT)
(74) Representative: Gervasi, Gemma, Dr.

(57) **Abstract**

The invention refers to an urinometer provided with an inlet opening which is connected to the catheter applied to a patient and to which a one-way valve is attached. The invention further refers to a one-way valve to be attached to said urinometer inlet opening, which is particularly suitable to avoid any risk that the urine stored inside the urinometer (and/or the urine vapour) goes back through the catheter up to the patient, or that it accidentally pours out of the urinometer.

## Description

### Field of the invention

The invention refers to an urinometer provided with an inlet opening which is connected to the catheter applied to a patient and to which a one-way valve is attached, and to a one-way valve to be attached to said urinometer inlet opening, said valve being particularly suitable to avoid any risk that the urine stored inside the urinometer (and/or the urine vapour) goes back through the catheter up to the patient, or that it accidentally pours out of the urinometer.

### Background art

Collecting and measuring devices for urine (called "urinometers 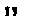 ) for catheterised patients are known and available on the market. These urinometers allow to measure the volume of the liquid excreted in a first period and in later prefixed periods and to separately sample the liquid excreted in the different periods in order to perform analysis of the same.

The urinometers are advantageously utilised to check the post-operative progress of patients. It is well known that it is very important to check for the full recovery of renal functions in the hours that immediately follow the operation, checking both the volume of excreted liquid and (by means of urine analysis) the expulsion of the remaining anaesthetic and of its metabolised products.

Urinometers are (or may be) advantageously used for monitoring the renal function in patients which are affected by diseases which strongly damage the renal function. In fact it is well known that belated "treatment" of a renal failure may cause the patient's death.

An efficient urinometer is described in the EP application No. 0 670144 by the present Applicants.

The use of an urinometer involves some risks for the catheterised patients which, as it is well known, are more subjected than other patients to infections of the urinary system.

This biggest risk can be due to the presence in the urinary system of pathogenic micro-organisms which are usually harmless in virtue of the immune defences. In a catheterised patient these defences can be weak and the urine collected in the urinometer is a favourable medium for the culture of pathogenic micro-organisms, which makes them "stronger". The pathogenic micro-organisms, when they are carried by the urine (or its vapours) through the catheter up to the urinary system, cause infections from which is often very difficult to recover.

The use of an urinometer can also involve risks for the sanitary staff attending patients having infective diseases such as AIDS, viral hepatitis, urogenital system infections, etc., in which the pathogenic agent can be excreted together with the urine. When an urinometer containing infected urine is separated from the catheter (for example to be transported to a laboratory or to be replaced with an empty urinometer or with an empty collecting bag) the possible pouring out of urine from the opening of the urinometer, to which the catheter is connected, can accidentally infect the sanitary staff.

In order to avoid any possible risk of infection, the Applicants have realised a very compact urinometer comprising a one-way valve placed at the inlet opening of the urinometer to which the catheter applied to the patient is connected, and a one-way valve (to be placed at said inlet opening of the urinometer) which avoids any risk that the urine contained in the urinometer goes through the catheter up to the patient or that the urine pours out from the urinometer.

### Summary of the invention

The objects of the present invention are an urinometer comprising a one-way valve placed at the inlet opening of the urinometer to which the catheter applied to the patient is connected, and said one-way valve particularly suitable to avoid any risk that the urine (or its vapours) contained in the urinometer goes through the catheter up to the patient or that the urine accidentally pours out from the urinometer.

### List of Drawings

The invention will be hereinafter described in greater detail with reference to a preferred embodiment, given as an example and illustrated in the drawings, in which:
- figure 1a schematically shows a side view of an urinometer according to the invention, comprising a one-way valve;
- figure 1b schematically shows a top view of the urinometer;
- figure 2 shows a side view of the body of the one-way valve;
- figure 3 shows a top view of the body of figure 2;
- figure 4 shows a section view of the body of figure 3 according to plane A-A;
- figure 5 shows a top view of the closure member to be applied to the one-way valve body.

In the drawings, the same reference numbers correspond to the same elements.

### Detailed description

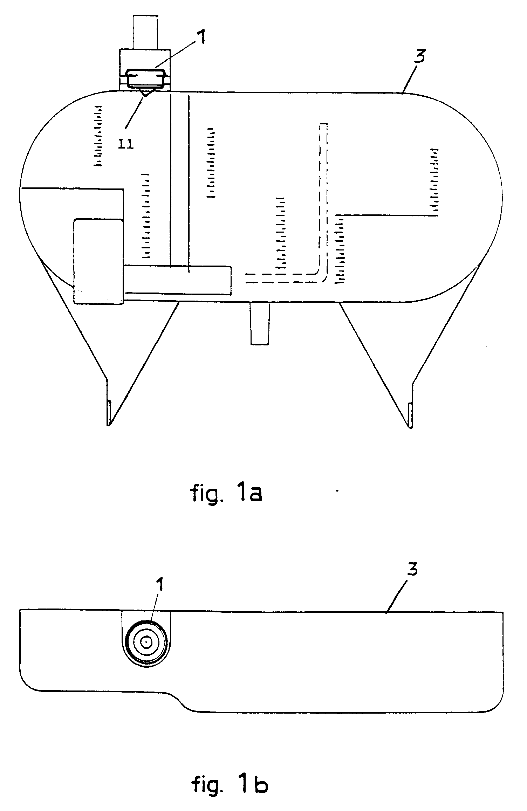

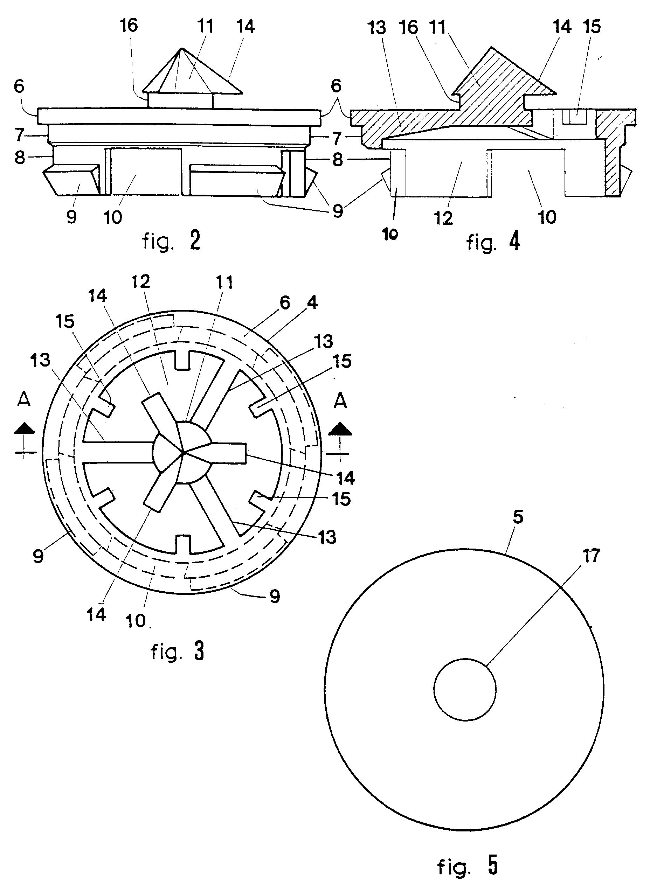

Figure 1a schematically shows a side view of an urinometer 3 according to the invention comprising a one-way valve 1 placed at the inlet opening of the urinometer 3. Said inlet opening is not shown in figures 1a, 1b because it is taken by the one-way valve 1. From figure 1a is evident that the substantially conical body 11 (figures 2-) of the one-way valve 1 placed at the inlet opening of the urinometer 3 is arranged inside the urinometer.

As schematically shown by figure 1a, the body of the urinometer 3 is divided in a plurality of inter-communicating chambers which can be emptied (one by one or simultaneously) by a tap placed at the bottom portion of the urinometer 3.

This kind of urinometer is known from the EP application No. 0 670144 in the name of the present Applicants and will not be further described.

In the known urinometers the catheter is placed at the extremity of a pipe (more or less long), which is integral with the inlet opening of the urinometer and which limits (or should limit) the risk that the urine accidentally pours out.

This pipe (the efficiency of which is doubtful when it is used as a safety means) was an obstacle to the realisation of urinometers as compact and handy as the urinometers of the present invention. In these urinometers the pipe has been removed and the inlet opening of the urinometer 3 is provided with a one-way valve 1 in order to avoid any risk of infection both for the patient and for the sanitary staff.

In the figures 2 to 5 a particularly advantageous preferred embodiment of the one-way valve 1 (comprising an hollow body 4 to which a closure member 5 is applied) will be described.

Figure 1b schematically shows an upper view of the urinometer 3 of fig.1a, in which the compactness of the urinometer is better shown; in figure 1b the one-way valve 1 housed in the inlet opening of the urinometer is shown. The inlet opening is not visible in figure 1b because it is taken by valve 1.

Figure 2 shows a side view of the hollow body 4 of valve 1, from which the closure member 5 (shown in figure 5) intended to close the inlet opening 2 of urinometer 3 has been removed.

Hollow body 4 has a substantially cylindrical shape, an internal cavity 12 (figure 3) and comprises three superimposed cylindrical zones: a first zone 6 having an external diameter bigger (and however not smaller) than the diameter of the inlet opening of the urinometer 3, a second zone 7 having an external diameter substantially of the same dimension (and however not bigger) as the diameter of said inlet opening and a third zone 8 (having a diameter smaller than the diameter of the second zone 7) on the external surface of which a plurality of hooked teeth 9 having a triangular section and cavities 10 alternate to teeth 9 are present.

On assembling valve 1 in the inlet opening of the urinometer 3, the extremity of the third cylindrical zone 8 of body 4 is inserted into said opening and an axial pressure is exerted. As a result of said axial pressure, the triangular teeth 9 enter the inlet opening and bend the portions of the third zone 8 by which they are supported (and which are flexible in virtue of the cavities 10). When the hooked teeth 9 pass the wall of the urinometer 3 over the inlet opening, the portions of zone 8 which support the hooked teeth 9 take the original position again and lock the valve to the urinometer inlet opening by a "click-on" motion.

In figure 2 a substantially conical body 11 is shown, which presents a narrow-section zone 16 the height of which is not lower than the thickness of the closure member 5 (figure 5) and which will be better described with reference to figures 3 and 4.

Figure 3 shows an upper view of the hollow body 4 of figure 2: in figure 3 the first cylindrical zone 6 of body 4 and the internal cavity 12 are shown. The substantially conical body 11 is carried by supports 13 integral with the zone 6 and is positioned in the centre of the cavity 12. A plurality of equally spaced radial lugs 14 protruding from the body 11 are present on the external surface of said body 11.

In figure 3, three supports 13 and three lugs 14 are shown. However, it is possible to use a different number of supports 13 and/or of lugs 14 without departing from the scope of the invention.

Furthermore, thickened zones (not shown in the figures in order to simplify the graphic representation) protruding from the narrow-section zone 16 are present at the extremities of the lugs 14.

Members 15 protruding towards the internal of the first zone 6 are provided, preferably at the same distance the one from the other, along the internal circumference of the first cylindrical zone 6. In figure 3 only one of the members 15 has been identified by the corresponding reference number in order to simplify the graphic representation.

In figure 3 the second and the third cylindrical zone (7,8) of the hollow body 4, the triangular hooked teeth 9 and the cavities 10 are indicated by broken lines. The corresponding references numbers have been omitted in order to simplify the graphic representation.

Figure 4 shows a sectional view of the hollow body 4 according the plane A-A of figure 3. In figure 4 the sections of the first and of the second cylindrical zones (6,7), of one of the supports 13, of the narrow-section zone 16, of the substantially conical body 11 and of one of the lugs 14, which is integral with the body 11, are shown.

In the right part of the figure, one of the triangular hooked teeth 9 and one of the protruding members 15, integral with the first cylindrical zone 6, are also shown. In the middle part of the figure one of the cavities 10 of the third cylindrical zone 8 is shown. In the left part of the figure one of the triangular hooked teeth 9 and one of the cavities 10 are shown.

From figure 4 it is evident that the extremities of the supports 13 placed at the opposite of the extremities integral with the first cylindrical zone 6 converge and are attached to the bottom of the narrow-section zone 16.

Figure 5 shows an upper view of the closure member 5 to be applied to the narrow-section zone 16 of the substantially conical body 11 (figure 2 and 4) in order to close the inlet opening of the urinometer 3. The closure member 5 consists of a thin disk of elastic material (latex, silicone or other equivalent, polymeric material) having a diameter bigger (and however not smaller) than the diameter of the above said inlet opening.

At the centre of the closure member 5 a hole 17 having a diameter not smaller than the diameter of the narrow-section zone 16 is present. The closure member 5 is assembled on zone 16 by forcing the substantially conical body 11 to pass through the hole 17 of the closure member 5.

Preferably, but not compulsory, the thickness of closure member 5 is between 1,7 and 5,7% of the closure member diameter.

The closure member 5 (very thin, flexible and light) is advantageous and efficient because, in resting conditions, it adheres to the wall of the urinometer 3 being pushed against the same by the lugs 14 and by thickened portions present at their extremities. In this way the closure member 5 closes the inlet opening (avoiding that the urine - and/or its vapours - pours out by capillarity) and let the urine to pass from the catheter to the urinometer 3. Possible back-flows of the urine towards the inlet opening press the closure member 5 against the edge of the inlet opening and against the protruding members 15. The members 15 give to the closure member 5 stiffness and tight-proof properties which avoid that the urine pours out through the inlet opening.

The described urinometer and one-way valve can be modified as suggested by day to day experience and by the evolution of the art without departing from the scope of the invention.

## Claims

1. Urinometer, characterised in that it comprises a one-way valve (1) placed at the inlet opening of said urinometer (3) to which the catheter applied to the patient is connected.

2. One-way valve for an urinometer as claimed in claim 1, characterised in that it comprises a substantially cylindrical hollow body (4) suitable to be inserted into said inlet opening of the urinometer, said hollow body (4) carrying a closure member (5) suitable to close said inlet opening of said urinometer (3).

3. One-way valve according to claim 2, characterised in that said hollow body (4) has a substantially cylindrical internal cavity (12) and comprises in combination:
- a first cylindrical zone (6) having a diameter not smaller than the diameter of said inlet opening of said urinometer (3);
- a second cylindrical zone (7) having a diameter not bigger than the diameter of said inlet opening of said urinometer (3);
- a third cylindrical zone (8) having a diameter smaller than the diameter of said second cylindrical zone (7) and including a plurality of triangular hooked teeth (9) and a plurality of cavities (10), alternate to said teeth (9), positioned on its external surface;
- a substantially conical body (11), which is placed at the centre of said internal cavity (12) of said hollow body (4) and which presents a narrow-section zone (16) the height of which is not smaller than the thickness of said closure member (5);
- a plurality of supports (13), the extremities of said supports being fixed to said first cylindrical zone (6) respectively at the base of said narrow-section zone (16); said hollow body (4) being further characterised in that
- said first, second and third cylindrical zones (6,7,8) are superimposed each to the other;
- a plurality of equally spaced, identical lugs (14) are present on the external surface of said substantially conical body (11) and radially protrude from said substantially conical body (11);
- identical members (15) are arranged along the internal circumference of said first cylindrical zone (6), the members (15) protruding inwards said first cylindrical zone (6).

4. One-way valve according to claim 3, characterised in that the diameter of said first cylindrical zone (6) is bigger than the diameter of said inlet opening of said urinometer (3).

5. One-way valve according to claim 3, characterised in that the diameter of said second cylindrical zone (7) is substantially equal to the diameter of said inlet opening of said urinometer (3).

6. One-way valve according to claim 3, characterised in that said members (15) are equally spaced along the internal circumference of said first cylindrical zone (6).

7. One-way valve according to claim 3, characterised in that said closure member (5) consists of a thin disk of elastic material having a diameter not smaller than the diameter of the inlet opening of said urinometer (3), said closure member (5) having a central hole (17) of a diameter not smaller than the external diameter of said narrow-section zone (16) of said substantially conical body (11).

8. One-way valve according to claim 7, characterised in that said diameter of the closure member (5) is bigger than the diameter of said inlet opening of said urinometer (3).

9. One-way valve according to claim 7, characterised in that the thickness of said closure member (5) is 1,7% to 5,7% of said diameter of said closure member (5).

10. One-way valve according to claim 3, characterised in that it is assembled in said inlet opening of said urinometer (3) by inserting the extremity of said third cylindrical zone (8) of the hollow body (4) in said inlet opening and by exerting an axial pressure on the valve; in that as a result of said axial pressure, said triangular hooked teeth (9) enter said inlet opening and bend said portions of said third cylindrical zone (8) by which they are supported; and in that said portions of said third cylindrical zone (8) supporting said triangular hooked teeth (9) take again the resting position when the triangular hooked teeth (9) pass over said inlet opening, thus fastening with a "click-on" motion the one-way valve (1) to said inlet opening of said urinometer (3).
